# EUROPEAN PATENT SPECIFICATION

(11) **EP 0 734 431 B1**
(45) Date of publication and mention of the grant of the patent: **25.02.1998**
(21) Application number: 95911553.6
(22) Date of filing: 13.12.1994
(51) Int. Cl.: C10M 133/12, C07C 291/02, C10M 169/04

(54) **LUBRICANT COMPOSITION CONTAINING A POLYMERIC ANTIOXIDANT**
EIN POLYMER-ANTIOXIDANT ENTHALTENDE SCHMIERMITTEL ZUSAMMENSETZUNG
COMPOSITION DE LUBRIFIANT CONTENAT UN ANTIOXYDANT POLYMERE

(30) Priority: 15.12.1993 US 166846; 09.12.1994 US 353416
(43) Date of publication of application: 02.10.1996
(73) Proprietor: EXXON RESEARCH AND ENGINEERING COMPANY, Florham Park, New Jersey 07932-0390 (US)
(72) Inventor: BERLOWITZ, Paul, J., East Windsor, NJ 08520 (US)
(74) Representative: Fletcher Watts, Susan J.
(86) International application number: US9414496
(87) International publication number: WO9517488

(56) References cited:
- US-A- 3 492 233
- US-A- 3 573 206
- US-A- 3 773 665

## Description

### BACKGROUND OF THE INVENTION

### Field of the Invention

This invention relates to aviation gas turbine lubricating oils.

### Description of the Related Art

Organic compositions such as mineral oils and lubricating compositions are subject to deterioration by oxidation and in particular are subject to such deterioration at high temperatures in the presence of air. This is particularly the case for lubricating oils used in jet aircraft where wide temperature ranges and extreme operating conditions are likely to be encountered. Proper lubricating of aircraft gas turbines, for example, requires ability to function at bulk oil temperatures as low as -54°C (-65°F) to as high as 232-260°C (450° - 500°F).

Oxidation stability of lubricating oils is further impaired by dissolution of metals under operation conditions. Dissolved metals apparently catalyze oxidative degradation of the lubricant, resulting in a shortening of its useful life.

Most lubricants contain additives to inhibit their oxidation. For example, U.S. Patent No. 3,773,665 discloses a lubricant composition containing an antioxidant additive mixture of dioctyl diphenylamine and a substituted naphthylamine.

U.S. Patent No. 3,759,996 discloses a process for oxidatively dimerizing N-phenyl-alpha-naphthylamines. The products produced by the process impart antioxidant properties, particularly to synthetic ester lubricants prepared from pentaerythritol and monocarboxylic acids.

U.S. Patent No. 3,573,206 discloses lubricating oils containing the reaction products from oxidation treatment of N-aryl naphthylamines, such as substituted N-phenyl naphthylamines (PNA), and diarylamines, such as substituted diphenyl amines (DPA), to form homo-oligomers of PNA and cross oligomers of DPA and PNA and a high percentage of unreacted of DPA and PNA. These reactions are desirably done in inert solvents such as aromatic hydrocarbon or ketones. These inert solvents avoid cross dehydrocondensation reactions described later.

It is also known that the treatment of various compounds with peroxide produces dehydrocondensation products having increased high temperature stability as antioxidants for lubricants over the monomeric components. U.S. Patent No. 3,492,233 discloses such a blend of a conventional polyester lubricating oil reacted in the presence of diaryl amines with certain organic peroxides to form dehydrocondensation products from esters and diaryl amines. These reactions require abstractable hydrogens on the polyester lubricants.

U.S. Patent No. 3,509,214 describes the high temperature air oxidation product or permanganate oxidation product from N-aryl naphthylamine or a combination thereof with diphenylamine.

It has now been discovered that the antioxidant properties of DPA and N-aryl naphthylamines, preferably PNA, reaction products employed in aviation gas turbine lubricating oils are improved by reacting controlled amounts of organic peroxides with specific molar ratios of DPA and PNA. These reaction products have higher concentrations of oligomer even in the presence of solvents with highly abstractable hydrogens. The composition is mainly homo-oligomers of DPA and cross oligomers of DPA and PNA.

### SUMMARY OF THE INVENTION

A lubricant composition which comprises a major amount of a synthetic ester aviation turbo oil and 0.1 to 10.0 weight percent, based on the total weight of the lubricant composition, of an antioxidant composition comprising the reaction product of:
(a) at least one N-aryl naphthylamine wherein said N-aryl naphthylamine has up to three independent alkyl and/or styryl substituted groups on each aryl ring, wherein said alkyl has from 1 to 20 carbon atoms and, said styryl is styryl or methyl substituted styryl;
(b) at least one diphenylamine wherein the phenyl groups are individually phenyl or phenyl having up to 3 alkyl or styryl substituent groups on each aryl ring, each alkyl having from 1 to 20 carbon atoms and the styryl being styryl or methyl substituted styryl; and
(c) an organic peroxide free radical source; wherein the mole ratio of said diphenylamine to said N-aryl naphthylamine is equal to or greater than about 1:1, the reaction is conducted from 70°C to 200°C and the reaction product comprises primarily a mixture of homo-oligomers of said diphenylamine and oligomers of said N-aryl naphthylamine and said diphenylamine.

### DETAILED DESCRIPTION OF THE INVENTION

The antioxidant of this invention is a mixture of homo-oligomers of diphenyl amines which are primarily dimers, or their alkylated derivatives and oligomers from substituted N-aryl naphthylamine, preferably PNA, and substituted diphenylamine.

The diphenyl amines (DPA) can be represented by the following general formula: 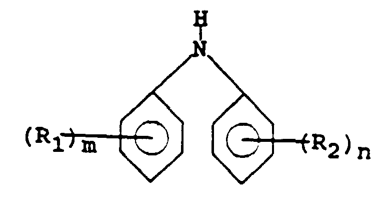 wherein each R₁ and R₂ independently are H, or branched or straight-chain C₁-C₂₀ alkyl radicals, or radicals such as styryl or methyl substituted styryl and preferably t-butyl or t-octyl radicals; and m and n each represent 0, 1, 2, or 3; preferably m and/or n represents 1.

The N-phenyl naphthylamine (PNA) can be represented by the following general formula: 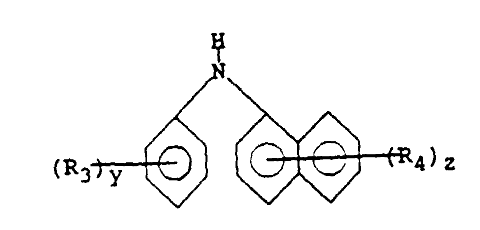 wherein R₃ and R₄ are independently C₁ to C₂₀ alkyl radicals or styryl or methyl styryl, desirably C₄-C₉ radicals and preferably t-butyl or t-octyl radicals; and y and z are independently 0, 1, 2, or 3, and preferably y and/or z represents 1.

It is theorized that the bonding between the DPA and PNA (or N-aryl naphthylamine) may occur between two nitrogen atoms, between a nitrogen atom in one aryl-naphthylamine or diphenylamine and a carbon atom in a naphthyl or other aryl radical, or between carbon atoms in two aryl rings from naphthyl or phenyl radicals. It is anticipated that most of the linkages between the DPA and PNA (or N-aryl naphthylamine) molecules are between nitrogen and carbon atoms in naphthyl or aryl substituents. The possible linkages are described in detail in U.S. Patent 3,509,214. The oligomers are believed to be very random in the order of DPA and PNA incorporation.

The antioxidant of the invention is made by reacting diphenylamine (DPA) with N-aryl-naphthylamine, preferably PNA, in the presence of peroxide at elevated temperature. In order to get high conversion of DPA and PNA to the desired oligomers, it is preferable that the DPA:PNA ratio be from 1.1:1 to 10:1; more preferably from 1.2:1 to 5:1; most preferably from 1.75:1 to 3:1, e.g., 2:1.

The reaction may be conducted in bulk or solution by heating the DPA, PNA, (hereinafter, where appropriate, PNA will refer to N-aryl naphthylamine or N-phenyl naphthylamine), and organic peroxide to temperatures from 70 to 200°C, desirably from 90 to 180°C, more desirably from 130 to 150°C, for from 30 minutes to 30 hours, desirably from 1 hour to 10 hours, and preferably from 2 to 6 hours. The reaction may be carried out under vacuum to remove volatiles from the decomposition of the organic peroxides. The DPA and PNA may be dissolved in suitable organic solvents such as aliphatic hydrocarbons, which can have abstractable hydrogens. The reaction may also be conducted in the presence of synthetic ester lubricants produced from condensation of monohydroxy alcohols and/or polyhydroxy alcohols with monocarboxylic or polycarboxylic acids.

These ester lubricants as disclosed in U.S. Patent 3,492,233, can become chemically bonded through dehydrocondensation reactions to the DPA, PNA, or oligomers thereof during the reaction of the DPA, PNA, and organic peroxides. However, with careful control of DPA to PNA ratios, the amount of peroxide used, and the reaction temperature; the amount of dehydrocondensation bond between the lubricant and amine is minimized.

Another useful solvent for the reaction of DPA, PNA, and organic peroxides are the alkane solvents having from 6 to 16 carbon atoms having linear, branched, or cyclic structure. These are also known to form dehydrocondensation products with these amines, but this reaction is limited in this disclosure by the reaction conditions. These solvents are also easily removed by volatilization.

Subsequent to the reaction of the DPA, PNA, and organic peroxides, it is desirable to raise the temperature to fully decompose the organic peroxides. This minimizes undesirable oxidation reactions later. Under optimized conditions as disclosed herein, most of the desired reactions have occurred prior to the decomposition step. It has been determined that no significant change in the molecular weight of the oligomers occurred during the decomposition of residual organic peroxides. Desirably, this is conducted at temperatures from 140 to 200°C, and desirably from 160-180°C for from 5 minutes to 2 hours, more desirably 30 minutes to 1 hour, and desirably at pressures below atmospheric pressure.

Reaction under the above-described conditions results in greater than 70 mole % of the DPA and PNA being in oligomeric forms of dimers or higher, desirably greater than 80 mole % in oligomeric forms of dimer or higher, more desirably greater than 90 mole % in oligomeric forms. of dimer or higher, and preferably greater than 95 mole % in oligomeric forms of dimer or higher. The residual portion of the DPA and PNA being primarily in monomer form, and less so dehydrocondensed with solvent or other molecules present.

Any organic peroxide may be used which has a half life of one hour at a temperature of from 70°C to 200°C. Desirably the half-life of one hour is at temperatures from 90 to 160°C, and preferably from 130 to 150°C. Included in this group are the peroxyesters and dialkyl peroxides, all of which are commercially available. The amount of peroxide used is desirably 0.5 to 3.0 moles/mole of the diaryl amines and is preferably from about 1.0 to 1.5, and most preferred from 1.1 to 1.3.

Included as peroxides are acyl peroxides of the formula 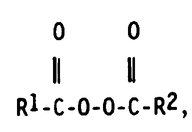 alkyl peroxides of the formula R¹-O-O-R², and peroxides 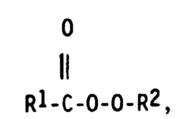 wherein R¹ and R² can be the same or different and alkyl, aromatic, alkyl substituted aromatic, or aromatic substituted alkyl groups having from 1 to 10 carbon atoms.

Suitable peroxides include t-amylperoxy-2-ethyl -hexanoate, t-butylperoxy-2-ethylhexanoate; t-butylperoxy-isobutyrate; t-butylperoxy-maleic acid; OO-t-butyl O-isopropyl monoperoxycarbonate; 2,5-dimethyl-2,5-di(benzoylperoxy)hexane; OO-t-butyl-O-(2-ethylhexyl)mono peroxycarbonate; OO-t-amyl O-(2-ethylhexyl)mono peroxycarbonate; t-butyl-peroxy acetate; t-amyl-peroxy-acetate; t-butylperoxy benzoate (t-butyl perbenzoate); t-amyl-peroxybenzoate (t-amyl perbenzoate) and di-t-butyl -diperoxyphthalate.

Suitable dialkyl peroxides include dicumyl peroxide; 2,5-dimethyl-2,5-di(t-butylperoxy) hexane; t-butyl cumyl peroxide; a-abis(t-butylperoxy)diisopropyl-benzene; di-t-butyl peroxide and 2,5-dimethyl-2,5-di(t-butylperoxy)hexyne-3. The most preferred peroxide is di-t-butyl peroxide.

The reaction products are desirably free of potassium permanganate or products of its reduction. These catalysts result in oligomeric products from diamines having reduced antioxidant effect. The reaction products desirably have 30 mole % or less, desirably 20 mole % or less, and preferably 10 mole % or less of the diaryl amines reacted into dehydrocondensation products with any solvent used for making the reaction products. Desirably the reaction products have at least 10 mole %, more desirably 15 to 30 mole %, and preferably 20-25 mole % of the diaryl amines in the form of homo-oligomers of DPA. Desirably at least 35 mole %, more desirably 40 mole %, and preferably 50 mole % of the diaryl amines are in the form of cross oligomers of DPA and PNA.

The antioxidants of this invention are useful in ester fluids including lubricating oils, particularly those ester fluids useful in high temperature avionic (turbine engines oils) applications.

The ester fluid lubricants which may be used with this invention are esters produced from monohydroxy alcohols and monocarboxylic acids, from polyhydroxy alcohols and monocarboxylic acids or from monohydroxy alcohols and dicarboxylic acids. Such esters are well known having been described, for example, in U.S. Patent No. 3,432,433. Each of the alcohols and acids used in preparing the ester may contain from 1 to 4 functional groups thereby producing mono-, di-, tri-, and tetraesters. Contemplated within this invention are esters of alcohols, diols, triols, and pentaerythritols, said alcohols or polyols having from 2 to 20 carbon atoms, and mono- and dicarboxylic acids having from 2 to about 20 carbon atoms and preferably 4 to 12.

The esters of this invention may include the monoesters from octyl acetate, decyl acetate, octadecyl acetate, methyl myristate, butyl stearate, methyl oleate, and the like and the polyesters from dibutyl phthalate, di-octyl adipate, di-2-ethylhexyl azelate, di-2-ethylhexyl sebacate, and the like.

Other esters include those produced from hindered or neopentyl alcohols, that is, those in which the beta carbon atom is completely substituted by other carbon atoms. These esters have the structure. 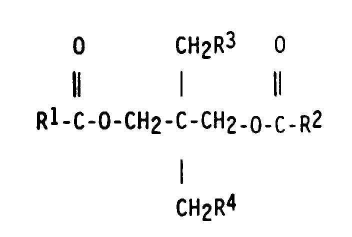 wherein each of R¹ and R² is alkyl or aryl of 1 to 19 carbon atoms and each of R³ and R⁴ is hydrogen, alkyl of 1 to 5 carbon atoms or 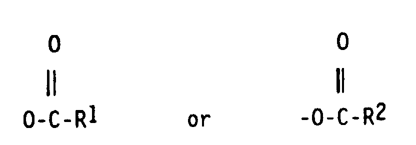 and each of the R¹ and R² groups and R³ and R⁴ groups may be the same or different. Such esters include 2,2-dimethylpropane-1,3-diol dipelargonate, trimethylolpropane trioctanoate, trimethylolpropane tridecanoate, trimethylolbutane trihexanoate, pentaerythrityl tetraoctanoate, neopentyl glycol and pentaerythrityl tetradodecanoate. Mixtures of acids may be used in producing the di-, tri- and tetraesters. For example, a preferred pentaerythritol ester contains a mixture of C₄ through C₁₀ carboxylic acids. The esters in accordance with this invention include any ester fluid having an abstractable hydrogen atom, although the preferred reaction conditions result in minimal dehydrocondensation between the polyesters and the amines.

The antioxidant of the invention may be employed in the lubricant composition at concentration levels ranging from 0.1 to 10.0, preferably 0.5 to 5.0, most preferably 1.5 to 2.0 weight percent based on the total weight of the lubricant composition. These weight percents are of the DPA, PNA, and oligomers thereof and do not include the synthetic ester lubricants even if they are used as a solvent for the reaction. In the case of synthetic ester lubricants coreacted with DPA and PNA, the weight percents recited above are the weight percents of DPA and PNA reactants in the final lubricant product.

The lubricant composition of the invention may also contain other additives to further improve the properties of the oil. Examples of other such additives are anti-corrosion agents, detergents, antifoamants, antiwear, extreme pressure additives, hydrolytic stability agents and other antioxidants.

The following examples will further illustrate the invention.

### EXAMPLES

### Example 1

An antioxidant product is prepared for comparison purposes by reacting p, p'- Di-t-octyl diphenylamine (DODPA) with N- (p-octylphenyl)-1-naphthylamine (OPANA) in a 1:1 molar ratio.

In a 5 liter, 3-neck flask equipped with a thermometer, an addition funnel and a distillation column, were placed 393 g (1 mole) of DODPA, 331.5 g (1 mole) of OPANA and 1 liter decane. The mixture was heated to 140°C under nitrogen and di-t-butyl peroxide (439 g, 3 mole) was added in portions. The reaction continued for 3 hours during which time t-butyl alcohol was collected through the distillation column. The reaction temperature was then raised to 170°C for 1 hour. More t-butyl alcohol was collected. Vacuum was then slowly applied to accelerate the distillation until 2 mm Hg was reached. Residue alcohol and decane were removed under vacuum. The vacuum was released under nitrogen and the mixture was poured into a cold container. The brittle solid produced was then grounded into yellow powder.

### Example 2

A preferred antioxidant of the invention was prepared by reacting DODPA with OPANA in a 2:1 molar ratio.

In a 5 liter, 3-neck flask equipped with a thermometer, an addition funnel and a distillation column, were placed 783 g (2 moles) of DODPA, 331.5 g (1 mole) of OPANA and 1114.5 g of an ester mixture consisting of a C₅ - C₁₀ acid pentaerithritol ester. The mixture was heated to 140°C under nitrogen and di-t-butyl peroxide (526.3 g, 3.6 mole) was added in portions over 45 minutes. The reaction continued for 3 hours during which time t-butyl alcohol was collected through the distillation column with a head temperature of 80-85°C. The color went from a fluorescent bluish color to a brown color. The reaction temperature was then raised to 170°C over a 1 hour period and was maintained there for 40 minutes. More t-butyl alcohol was collected. The vacuum was then slowly applied to accelerate the distillation until a pressure of 2 mm Hg was reached. The reaction product was held under those conditions 20 minutes to remove all residue alcohol. The vacuum was released under nitrogen and the mixture was cooled down. The reaction product was then collected as a 50% active antioxidant in the ester.

### Example 3

A preferred antioxidant of the invention was prepared by reacting DODPA with OPANA in a 3:1 molar ratio. In a 250 ml 3-neck round bottom flask equipped with a thermometer, addition funnel and magnetic stirrer, 23.6 g (0.06 mole) of DODPA and 6.63 g (0.02 mole) of OPANA were mixed with 30.2 g of the ester of Example 2. While heating to 140°C under nitrogen, t-butyl peroxide (14.04 g, 0.096 mole) was added in portions during a half-hour period. The reaction was stirred at 140°C for a total of 7 hours, then at 170°C for 45 minutes. Vacuum (2mm Hg) was applied at the end for 20 minutes at 170°C.

### Example 4

The reaction products of Example 1 and 3 were evaluated in oxidation corrosion stability (OCS) tests in the presence of various metals at different temperatures. Commercial product A shown in Table I represents a commercial material made from a mixture having at least a 1:2 molar ratio of DODPA:OPANA being present at 2.0 wt% in a synthetic ester lubricant. The products of Examples 1 and 3 were evaluated at 2 wt% in an ester lubricant. The OCS test is the exposure of a synthetic ester lubricant (condensation product of pentaerythritol and mixed C₄-C₉ carboxylic acid) to temperature of 400 or 425°C for 72 hours while metals are present. It determines the ability of the antioxidants to inhibit oxidation of the lubricant and formation of acid species. It measures the change in viscosity of the lubricant as a % of the initial viscosity and the change in total acid number (ΔTAN). The viscosity is measured as kinematic viscosity at 37,8°C (100°F). The results in Table I below show the change in viscosity (Δ vis %) and change in total acid number (Δ TAN) for each example with the different metals present. The Δ TAN is calculated from the moles of additional base required to or titrate to neutral 100 g of sample multiplied times 561.

**TABLE I**

| Experiment | Measure | Example 1 | Example 3 | Commercial Material A |
|---|---|---|---|---|
| OCS 204,4°C (400°F) | Δ Vis% | 5.5 | 3.9 | 10.0 |
| Cu.MG.Fe.Al.Ag | Δ TAN | 0.15 | 0.06 | 0.51 |
| OCS 218,5°C (425°F) | Δ Vis% | 30.5 | 13.3 | 36.0 |
| Cu.Mg.Fe.Al.Ag | Δ TAN | 5.7 | 1.99 | 4.7 |
| OCS 218,5°C (425°F) | Δ Vis% | 26.95 | 9.7 | 20.84 |
| Tl.Ti.Fe.Al.Ag | Δ TAN | 11.7 | 0.86 | 3.35 |
| OCS 232°C (450°F) | Δ Vis% | Not Available | 72.5 | 127,070 |
| Cu.Mg.Fe.Al.Ag | Δ TAN | Not Available | 4.8 | 8.01 |

The data in Table I shows that the material of Example 3 using a 3:1 molar ratio of DODPA to OPANA, lower temperature and a controlled amount of peroxide performs better in the OCS test than the Commercial Material A, which as at least a 1:2 molar ratio of DODPA to OPANA and the antioxidant of Example 1 which has a 1:1 molar ratio of DODPA to OPANA.

### Example 5

The materials of Examples 1, 3, and Comparison Material A were also tested in the U.S. Navy Vapor Phase Coker Test. This test is fully described in publication NAPTC-PE-71 of the Naval Air Propulsion Test Center. The test is designed to simulate part of a gas turbine engine where hot surfaces are contacted by oil mists or vapors. It consists of a round bottom flask held at 204,4°C (400°F) into which 765 CC/minute (0.027 scfm) of dry air is bubbled for 18 hours. The vapor and mist formed from the bubbling air flow up into a metal tube which is in an oven held at 371,1°C (700°F). The tube is tarred before the test, and weighed afterwards to measure the mist and vapor deposit formed. A low value in this test is desirable as it indicates a lubricant with minimized tendency to form undesirable vapor/mist deposit in gas turbine engines. The average test results for the product of Example 1 were 180 mg; the test results for the product of Example 3 were 138 mg, and the test results from Commercial Material A were 295 mg. These tests indicate that a preferred antioxidant of the invention (Example 3) produces less of the undesirable deposits during high temperature use than related antioxidants (Example 1 and Commercial Material A).

### Example 6

To study the effect of reaction conditions and DODPA:OPANA ratio on the performance of oligomeric amine reaction products, several examples from U.S. Patent 3,573,206 were made replacing N-phenyl-2-naphthylamine with the more effective t-octyl N-phenyl-1-naphthylamine and replacing diphenylamine with the more effective p,p'-di-t-octyl-diphenylamine used in this application. Example B is from Example 5 of U.S. Patent 3,573,206 and Example C is from Example 9 of this patent. Both examples of said patent use the more effective alkyl substituted amines so as to be more comparable to Examples 2 and 3 of this disclosure. Example B used only OPANA, while Example C used an equimolar blend of DODPA and OPANA. Both Examples B and C used potassium permanganate to cause oxidation. As disclosed in the patent, the unreacted amines were greater than 40 weight percent of the reaction products using the permanganate oxidation technology.

In Example 5 of U.S. Patent 3,573,206, about one-half of the reaction product was dimer and one-half was unreacted. In Example 9 of the patent, about 44% of the reaction product was the starting materials, about 35% was the dimer, about 15% was a desirable cross-oligomer, and about 5% was an unidentified side product.

The antioxidants of Examples 2, 3, B and C were subjected to the OCS Test at 218,5°C (425°F) for 72 hours. The results are given below in Table II.

**TABLE II**

| OCS Test at 218,5°C (425°F), 72 Hours | | | |
|---|---|---|---|
| DODPA:OPANA (2 wt. % in ester) | | Δ Vis % | Δ TAN |
| Example 2 | 2:1 | 18.8 | 5.65 |
| Example B | 0:1 | 41.5 | 7.15 |
| Example C | 1:1 | 41.0 | 12.4 |
| Example 3 | 3:1 | 24.3 | 4.39 |

Table II shows that the compositions of Examples 2 and 3 perform better at prevention of oxidative changes in the lubricant compositions than do Examples B and C made with permanganate oxidation. This shows that the ratio of DODPA:OPANA and the reaction conditions such as peroxides versus potassium permanganate have an observable effect on the performance of the reaction products.

### Example 7

To study the effect of dehydrocondensation between the solvent and the diamines, Examples D and E were prepared. Example D was made with a mole ratio DODPA:OPANA of 1:1 in 1-decane solvent with enough t-butyl peroxide to cause greater than 90 mole % of the diaryl amines to go through dehydrocondensation with the 1 decane. Example E was made with a mole ratio DODPA:OPANA of 2:1 in a pentaerythritol ester of C₅-C₉, linear and branched fatty acids. Example E was made with t-butyl peroxide in a similar fashion as in Example 1 of U.S. Patent 3,492,233, where about 70 mole % of the diarylamine was dehydrocondensed with the ester. Table III shows the results of using these antioxidants in oxidation stability tests.

**TABLE III**

| OCS Tests @ 218,5°C (425°F), 72 Hours Al, Ti, Ag, Steel Present | | | |
|---|---|---|---|
| | DODPA:PANA (2 Wt. %) | Δ Vis % | Δ TAN |
| Example 2 | 2:1 | 27.8 | 2.41 |
| Example D | 1:1 | 78.0 | 11.26 |
| Example E | 2:1 | 68.3 | 10.71 |

As can be seen from Table III, the amine antioxidants which have dehydrocondensed with the solvent (Examples D and E) are dramatically less efficient antioxidants in terms of Δ Vis % or Δ TAN.

Example E with the higher more preferred DODPA ratio produced slightly better results but was not comparable to Example 2 with the same DODPA:OPANA ratio.

### Example 8

The following samples were prepared for comparison purposes by reacting DODPA and OPANA in the presence of various oxidizing agents. All reactions were carried out using the laboratory equipment and general procedures described in the prior examples.
Sample 8A - 1.0 moles of DODPA were reacted with 1.0 moles of OPANA in the presence of 1.25 moles of t-butylperoxide and the C₅-C₁₀ acid pentaerithritol (Ester) of Example 2 for 2 hours 140°C followed by 1 hour at 170°C to remove residual peroxide. The amount of Ester employed was equal to one half of the total weight of the reaction mixture including the Ester (50% Ester). Sample 8A is a product of the invention.
Sample 8B - 1.5 moles of DODPA were reacted with 1.0 moles of OPANA in the presence of 1.25 moles of t-butylperoxide and 50% Ester for 2 hours at 140°C followed by 1 hour at 170°C to remove residual peroxide. Sample 8B is a product of the invention.
Sample 8C - 0.16 moles of DODPA and 0.08 moles of OPANA were reacted in the presence of 0.276 moles of t-butylperoxide and 50% Ester for 2 hours at 140°C followed by 45 minutes at 170°C to remove residual peroxide. Sample 8C is a product of the invention.
Sample 8D - 1.0 moles of DODPA were reacted with 1.0 moles of OPANA in the presence of 0.5 moles KMnO₄ for 72 hours in the manner of Example 9 of U.S. Patent No. 3,573,206. The resultant product was diluted with 50% Ester. Sample 8D is not a product of the invention.
Sample 8E - 1.0 moles of DODPA were reacted with 1.0 moles of OPANA in the presence of 1.25 moles of KMnO₄ for 3 hours at 150°C. The resultant product was diluted with toluene, filtered, dried and then diluted with 50% Ester. Sample 8E is not a product of the invention.
Sample 8F - 1.0 moles of DODPA were reacted with 1.0 moles of OPANA in the presence of air for 72 hours at 250°C in a manner similar to Examples 2 and 3 of U.S. Patent No. 3,573,206. The resultant product was diluted with 50% Ester. Sample 8F is not a product of the invention.
Sample 8G - 1.0 moles of DODPA were reacted with 1.0 moles of OPANA in the presence of 30% H₂O₂ (1.2 moles) for 3 hours at 150° followed by 1 hour at 170°C to remove residual peroxide. The resultant product was diluted with 50% Ester. Sample 8G is not a product of the invention.
Sample 8H - 1.0 moles of DODPA were reacted with 1.0 moles of OPANA in the presence of 1.25 moles of m-chloroperbenzoic acid for 2 hours at 150°C followed by 1 hour at 170°C. The resultant product was diluted with toluene, filtered, washed with 10% sodium carbonate, dried and then diluted with 50% Ester. Sample 8H is not a product of the invention.
Sample 8I - 1.0 moles of DODPA were reacted with 1.0 moles of OPANA in the presence of 1.5 moles of MnO₂ for 4 hours at 100-140°C. The resultant product was diluted with toluene, filtered, dried and then diluted with 50% Ester. Sample 8I is not a product of the invention.
Sample 8J - 1.0 moles of DODPA were reacted with 1.0 moles of OPANA in the presence of 1.25 moles of PbO₂ for 5 hours at 120-200°C. The resultant product was diluted with toluene, filtered, dried and then diluted with 25% Ester. Sample 8J is not a product of the invention.

Samples 8A - 8J were evaluated in the OCS Test for 72 hours at 204,4°C (400°F) and for 72 hours at 218,5°C (425°F) in the presence of copper, magnesium, stainless steel, aluminum and silver. In all cases 4.0 wt.% of the Sample was added to the test synthetic ester, except 2.0 wt.% of Sample 8J was added. The results are given below in Tables IV and V.

**TABLE IV**

| OCS Test Results at 204,4°C (400°F) for 72 Hours | | | |
|---|---|---|---|
| Sample | Δ Vis | Δ TAN | Cu Corrosion |
| 8A | 12.41 | 0.52 | -0.05 |
| 8B | 12.13 | 0.47 | -0.06 |
| 8C | 12.04 | 0.41 | -0.06 |
| 8D | 18.26 | 0.65 | -0.08 |
| 8E | 14.65 | 0.66 | -0.08 |
| 8F | 151.5 | 7.0 | -0.05 |
| 8G | 16.43 | 0.67 | -0.05 |
| 8H | 18.70 | 1.12 | -0.12 |
| 8I | 17.34 | 0.84 | -0.09 |
| 8J | 17.38 | -0.11* | -0.03 |

| | | | |
|---|---|---|---|
| * Large loss of magnesium metal was noted. | | | |

**TABLE V**

| OCS Test Results at 218,5°C (425°F) for 72 Hours | | | |
|---|---|---|---|
| Sample | Δ Vis | Δ TAN | Sludge mg |
| 8A | 26.83 | 2.23 | 0.0 |
| 8B | 28.19 | 0.96 | 0.0 |
| 8C | 26.51 | 1.44 | 0.1 |
| 8D | 52.48 | 5.36 | 1.6 |
| 8E | 38.10 | 3.93 | 0.8 |
| 8F | 79.60 | 7.78 | 3.8 |
| 8G | 46.99 | 1.70 | 1.6 |
| 8H | 34.14 | 1.74 | 0.7 |
| 8I | 38.93 | 4.20 | 0.8 |
| 8J | 31.92 | -0.41* | 6.5 |

| | | | |
|---|---|---|---|
| * Large loss of magnesium metal was noted. | | | |

The results given in Tables IV and V show that the use of organic peroxides to prepare polymeric antioxidants from diphenyl amine and aryl naphthylamine results in an improved product as compared to those materials made in the presence of other oxidizing agents.

## Claims

1. A lubricant composition which comprises a major amount of a synthetic ester aviation turbo oil and 0.1 to 10.0 weight percent, based on the total weight of the lubricant composition, of an antioxidant composition comprising the reaction product of:
(a) at least one N-aryl naphthylamine wherein said N-aryl naphthylamine has up to three independent alkyl and/or styryl substituted groups on each aryl ring, wherein said alkyl has from 1 to 20 carbon atoms and, said styryl is styryl or methyl substituted styryl;
(b) at least one diphenylamine wherein the phenyl groups are individually phenyl or phenyl having up to 3 alkyl or styryl substituent groups on each aryl ring, each alkyl having from 1 to 20 carbon atoms and the styryl being styryl or methyl substituted styryl; and
(c) an organic peroxide free radical source; wherein the mole ratio of said diphenylamine to said N-aryl naphthylamine is equal to or greater than about 1:1, the reaction is conducted from about 70°C to 200°C and the reaction product comprises primarily a mixture of homo-oligomers of said diphenylamine and oligomers of said N-aryl naphthylamine and said diphenylamine.

2. The lubricant of claim 1 wherein (i) the diphenyl amines (DPA) is represented by the following general formula: 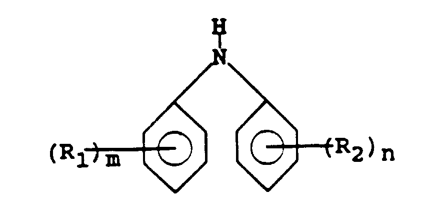 wherein each R₁ and R₂ independently are H, or branched or straight - chain C₁-C₂₀ alkyl radicals and m and n each represent 0, 1, 2 or 3 and (ii) the N-aryl naphthylamine is N-phenyl naphthylamine (PNA) represented by the general formula: 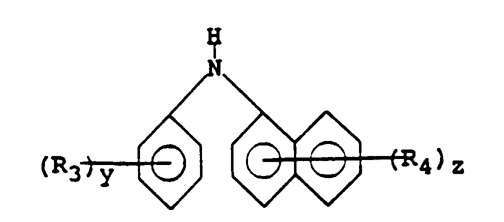 wherein R₃ and R₄ are independently C₁ to C₂₀ branched or straight - chain alkyl radicals and y and z represent 0, 1, 2, or 3 and the mole ratio of DPA to PNA is from 1.1:1 to 10:1.

3. The lubricant of claim 1 or 2 wherein the peroxide type free radical source is present from 0.5 to 3.0 moles per mole of combined DPA and PNA and wherein the mole ratio of DPA to PNA is from 1.2:1 to 5:1.

4. The lubricant of any preceding claim wherein the molar ratio of said organic peroxide to the combined moles of DPA and PNA is from 1:1 to 1:5 and wherein the reaction is substantially free of permanganate.

5. The lubricant of any preceding claim wherein the mole ratio of DPA to PNA is from 1.75:1 to 3:1.

6. The lubricant of any preceding claim wherein R₁, R₂, R₃ and R₄ are independently C₄ to C₈ alkyl radicals and m, n, y and z are 1.

7. The lubricant of claim 6 wherein R₁, R₂, R₃ and R₄ are independently t-butyl or t-octyl radicals.

8. The lubricant of claim 6 wherein R₁, R₂, R₃ and R₄ are t-octyl radicals.

9. The lubricant of any preceding claim wherein the reaction product is at least 30 wt.% of oligomers containing at least one DPA unit and one PNA unit.

10. The lubricant of any preceding claim wherein the amount of the antioxidant composition is 0.5 to 5.0 weight percent based on the total weight of the lubricant and wherein the antioxidant composition comprises the reaction product of p,p'-di-t-octyl diphenylamine (DODPA) with N-(p-octylphenyl)-1-naphthylamine (OPANA).

## Patentansprüche

1. Schmiermittelzusammensetzung, die eine größere Menge eines synthetischen Esterflugturboöls und 0,1 bis 10,0 Gew.-%, bezogen auf das Gesamtgewicht der Schmiermittelzusammensetzung, einer Antioxidanszusammensetzung, die das Reaktionsprodukt von:
(a) mindestens einem N-Arylnaphthylamin, in dem das N-Arylnaphthylamin bis zu 3 unabhängige Alkyl- und/oder Styrylsubstituentengruppen an jedem Arylring aufweist, wobei die Alkylgruppe 1 bis 20 Kohlenstoffatome besitzt und das Styryl Styryl oder methylsubstituiertes Styryl ist,
(b) mindestens einem Diphenylamin, in dem die Phenylgruppen individuell Phenyl oder Phenyl mit bis zu 3 Alkyl- oder Styrylsubstituentengruppen an jedem Arylring sind, wobei jedes Alkyl 1 bis 20 Kohlenstoffatome besitzt und das Styryl Styryl oder methylsubstituiertes Styryl ist, und
(c) einer organischen Peroxidfreiradikalquelle umfaßt,
wobei das Molverhältnis des Diphenylamins zu dem N-Arylnaphthylamin gleich oder größer als etwa 1 : 1 ist, die Reaktion bei etwa 70 °C bis 200 °C durchgeführt wird und das Reaktionsprodukt primäre eine Mischung aus Homooligomeren des Diphenylamins und Oligomeren des N-Arylphaphthylamins und des Diphenylamins umfaßt.

2. Schmiermittel nach Anspruch 1, bei dem (i) die Diphenylamine (DPA) durch die folgende allgemeine Formel repräsentiert sind: 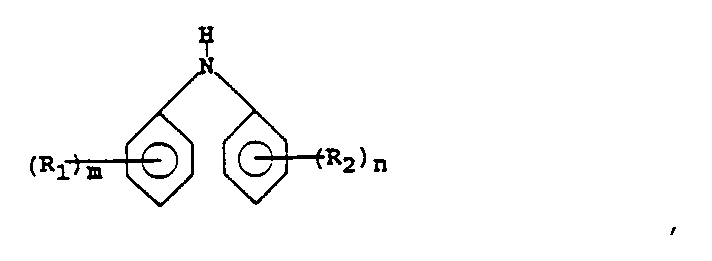 in der jedes R₁ und R₂ jeweils unabhängig H oder verzweigte oder geradkettige C₁- bis C₂₀-Alkylreste sind und m und n jeweils 0, 1, 2 oder 3 sind, und (ii) das N-Arylnaphthylamin N-Phenylnaphthylamin (PNA) ist, das durch die allgemeine Formel: 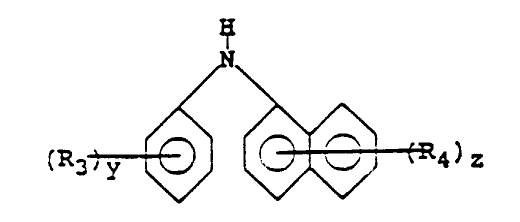 repräsentiert ist, in der R₃ und R₄ jeweils unabhängig verzweigte oder geradkettige C₁- bis C₂₀-Alkylreste sind und y und z 0, 1, 2 oder 3 sind, wobei das Molverhältnis von DPA zu PNA 1,1 : 1 bis 10 : 1 beträgt.

3. Schmiermittel nach Anspruch 1 oder 2, bei dem die freie Radikalquelle vom Peroxidtyp mit 0,5 bis 3,0 Molen pro Mol von kombiniertem DPA und PNA vorhanden ist und wobei das Molverhältnis von DPA zu PNA 1,2 : 1 bis 5 : 1 beträgt.

4. Schmiermittel nach einem der vorhergehenden Ansprüche, bei dem das Molverhältnis des organischen Peroxids zu den kombinierten Molen von DPA und PNA 1 : 1 bis 1 : 5 beträgt und bei dem die Reaktion im wesentlichen frei von Permanganat ist.

5. Schmiermittel nach einem der vorhergehenden Ansprüche, bei dem das Molverhältnis von DPA zu PNA 1,75 : 1 bis 3 : 1 beträgt.

6. Schmiermittel nach einem der vorhergehenden Ansprüche, bei dem R₁, R₂, R₃ und R₄ jeweils unabhängig C₄- bis C₈-Alkylreste sind und m, n, y und z 1 sind.

7. Schmiermittel nach Anspruch 6, bei dem R₁, R₂, R₃ und R₄ jeweils unabhängig t-Butyl- oder t-Octylreste sind.

8. Schmiermittel nach Anspruch 6, bei dem R₁, R₂, R₃ und R₄ t-Octylreste sind.

9. Schmiermittel nach einem der vorhergehenden Ansprüche, bei dem das Reaktionsprodukt mindestens 30 Gew.-% Oligomere sind, die mindestens eine DPA-Einheit und eine PNA-Einheit enthalten.

10. Schmiermittel nach einem der vorhergehenden Ansprüche, bei dem die Menge der Antioxidanszusammensetzung 0,5 bis 5,0 Gew.-%, bezogen auf das Gesamtgewicht des Schmiermittels, ist und bei dem die Antioxidanszusammensetzung das Reaktionsprodukt von p,p'-Di-t-octyldiphenylamin (DODPA) mit N-(p-Octylphenyl)-1-naphthylamin (OPANA) ist.

## Revendications

1. Composition lubrifiante qui comprend une quantité majeure d'une huile turbo d'aviation formé d'un ester synthétique et 0,1 à 10,0% en poids, sur la base du poids total de la composition lubrifiante, d'une composition antioxydante comprenant le produit réactionnel :
(a) d'au moins une N-arylnaphtylamine qui a jusqu'à trois groupes substituants alkyle et/ou styryle indépendants sur chaque noyau aryle, ledit groupe alkyle ayant 1 à 20 atomes de carbone et ledit groupe styryle étant un groupe styryle ou un groupe styryle à substitution méthyle,
(b) d'au moins une diphénylamine dans laquelle les groupes phényle sont individuellement un groupe phényle ou un groupe phényle ayant jusqu'à trois groupes substituants alkyle ou styryle sur chaque noyau aryle, chaque groupe alkyle ayant 1 à 20 atomes de carbone et le groupe styryle étant un groupe styryle ou un groupe styryle à substitution méthyle, et
(c) d'une source de radicaux libres à base de peroxyde organique, dans laquelle le rapport molaire de ladite diphénylamine à ladite N-arylnaphtylamine est égal ou supérieur à environ 1:1, la réaction étant réalisée à une température d'environ 70 à 200°C et le produit réactionnel comprenant principalement un mélange d'homo-oligomères de ladite diphénylamine et d'oligomères de ladite N-arylnaphtylamine et de ladite diphénylamine.

2. Lubrifiant selon la revendication 1, dans lequel (i) la diphénylamine (DPA) est représentée par la formule générale suivante : 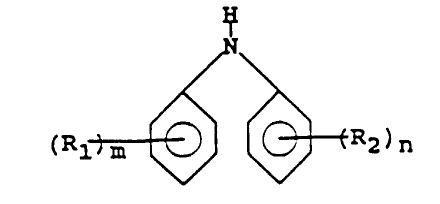 dans laquelle chaque groupe R₁ et R₂ est indépendamment H ou des radicaux alkyle à chaîne ramifiée ou droite en C₁-C₂₀ et m et n représentent chacun 0, 1, 2 ou 3 et (ii) la N-arylnaphtylamine est la N-phénylnaphtylamine (PNA) représentée par la formule suivante : 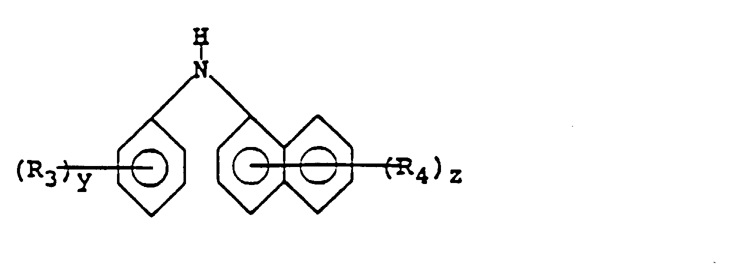 dans laquelle R₃ et R₄ sont indépendamment des radicaux alkyle à chaîne ramifiée ou droite en C₁-C₂₀, y et z représentent 0, 1, 2 ou 3 et le rapport molaire de la DPA à la PNA est de 1,1:1 à 10:1.

3. Lubrifiant selon la revendication 1 ou 2, dans lequel la source de radicaux libres à base de peroxyde est présente à raison de 0,5 à 3,0 moles par mole de DPA et de PNA combinées et dans lequel le rapport molaire de la DPA à la PNA est de 1,2:1 à 5:1.

4. Lubrifiant selon l'une quelconque des revendications précédentes, dans lequel le rapport molaire dudit peroxyde organique aux moles combinées de DPA et de PNA est de 1:1 à 1:5 et dans lequel la réaction est sensiblement exempte de permanganate.

5. Lubrifiant selon l'une quelconque des revendications précédentes, dans lequel le rapport molaire de la DPA à la PNA est de 1,75:1 à 3:1.

6. Lubrifiant selon l'une quelconque des revendications précédentes, dans lequel R₁, R₂, R₃ et R₄ sont indépendamment des radicaux alkyle en C₄ à C₈ et m, n, y et z sont égaux à 1.

7. Lubrifiant selon la revendication 6, dans lequel R₁, R₂, R₃ et R₄ sont indépendamment des radicaux t-butyle ou t-octyle.

8. Lubrifiant selon la revendication 6, dans lequel R₁, R₂, R₃ et R₄ sont des radicaux t-octyle.

9. Lubrifiant selon l'une quelconque des revendications précédentes, dans lequel le produit réactionnel est formé d'au moins 30% en poids d'oligomères contenant au moins une unité DPA et une unité PNA.

10. Lubrifiant selon l'une quelconque des revendications précédentes, dans lequel la quantité de la composition antioxydante est de 0,5 à 5,0% en poids sur la base du poids total du lubrifiant et dans lequel la composition antioxydante comprend le produit réactionnel de la p,p'-di-t-octyldiphénylamine (DODPA) avec la N-(p-octylphényl)-1-naphtylamine (OPANA).
